# EUROPÄISCHE PATENTSCHRIFT

(11) **EP 2 059 169 B1**
(45) Veröffentlichungstag und Bekanntmachung des Hinweises auf die Patenterteilung: **19.09.2012**
(21) Anmeldenummer: 07819991.6
(22) Anmeldetag: 30.08.2007
(51) Int. Cl.: A61B 5/15

(54) **MEDIZINISCHES HILFSMITTEL**
MEDICAL CONTRIVANCE
DISPOSITIF MÉDICAL

(30) Priorität: 04.09.2006 EP 06120063
(43) Veröffentlichungstag der Anmeldung: 20.05.2009
(73) Patentinhaber: F.Hoffmann-La Roche AG, 4070 Basel (CH); Roche Diagnostics GmbH, 68305 Mannheim (DE)
(72) Erfinder: LIST, Hans, 64754 Hesseneck-Kailbach (DE)
(74) Vertreter: Stößel, Matthias
(86) Internationale Anmeldenummer: PCT/EP2007/059024
(87) Internationale Veröffentlichungsnummer: WO 2008/028856

(56) Entgegenhaltungen:
- EP-A1- 1 508 304
- WO-A-2004/075760
- GB-A- 1 099 920

## Beschreibung

Die Erfindung bezieht sich auf ein medizinisches Hilfsmittel mit einer Lanzette und auf einen Sensor mit einem Testelement, wobei die Lanzette oder das Testelement zumindest teilweise von einer (bevorzugt flexiblen) Verpackung umschlossen sind.

Die Untersuchung von Blutproben oder von interstitieller Flüssigkeit ermöglichen in der klinischen Diagnostik das frühzeitige und zuverlässige Erkennen von pathologischen Zuständen sowie die gezielte und fundierte Kontrolle von Körperzuständen. Die medizinische Diagnostik setzt stets die Gewinnung einer Probe aus Blut oder interstitieller Flüssigkeit des zu untersuchenden Individuums voraus.

Zur Gewinnung der Probe kann die Haut z.B. an der Fingerbeere oder dem Ohrläppchen der zu untersuchenden Person mit Hilfe einer sterilen, scharfen Lanzette perforiert werden, um so einige wenige Mikroliter Blut oder weniger für die Analyse zu gewinnen. Insbesondere eignet sich diese Methode für die Analyse einer Probe, die unmittelbar nach der Probengewinnung durchgeführt wird.

Vor allem im Bereich des so genannten "Home-Monitoring", also dort, wo medizinische Laien selbst einfache Analysen des Bluts oder der interstitiellen Flüssigkeit durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte (so genannte Stechhilfen), angeboten, die eine möglichst schmerzarme und reproduzierbare Probengewinnung ermöglichen. Solche Lanzetten und Geräte (Stechhilfen) sind z.B. Gegenstand von WO-A 98/48695, US 4,442,836 oder US 5,554,166.

Die eigenhändige Blutzuckerbestimmung ist heute eine weltweit verbreitete Methode in der Diabetes-Kontrolle. Blutzuckergeräte im Stand der Technik bestehen aus einem Analysegerät, in das ein Testelement (Teststreifen) eingeführt wird. Das Testelement wird mit einem Tropfen einer Probe in Kontakt gebracht, der zuvor mittels einer Stechhilfe z.B. aus der Fingerbeere gewonnen wurde.

Weiterhin sind aus dem Stand der Technik zahlreiche Testelemente und Sensoren mit derartigen Testelementen bekannt, welche einsetzbar sind, um die Konzentration mindestens eines Analyten (beispielsweise Glucose, Lactat oder Cholesterin) in der flüssigen Probe, insbesondere in Blut oder interstitieller Flüssigkeit, zu bestimmen. Diese Testelemente oder Sensoren können beispielsweise auf elektrochemischen Messmethoden beruhen, wie beispielsweise in US 5,286,362 beschrieben. Andere Testelemente und Sensoren beruhen beispielsweise auch eine optischen Nachweis des mindestens einen Analyten. So kann der Analyt insbesondere mit einem auf oder in dem Testelement enthaltenen Nachweisreagens reagieren, wobei das Testelement (beispielsweise ein Testfeld des Testelements) mindestens eine messbare optische Eigenschaft (z.B. eine Farbe, eine Fluoreszenz, eine Reflektivität oder eine Kombination dieser Eigenschaften) ändert. Die Änderung dieser Eigenschaft kann optisch nachgewiesen werden, um daraus die Konzentration des mindestens einen Analyten zu bestimmen. Ausführungsbeispiele derartiger, im Folgenden auch als "optische Testelemente" bezeichneten Testelemente bzw. Sensoren sind beispielsweise aus CA 2,050,677 bekannt.

Weiterhin sind auch Testelemente und Sensoren bekannt, welche derart magaziniert sind, dass ein Magazin mehrere derartiger Testelemente umfasst. Beispielsweise können mehrere Testelemente oder Sensoren auf einem Analyseband aufgenommen sein. Beispiele derartiger Analysebänder mit mehreren Testfeldern sind in EP 0 299 517 B1 offenbart.

Weiterhin sind auch Sensoren und Testelemente bekannt, welche nicht für einen einmaligen, kurzen Kontakt mit der Probe konzipiert sind, sondern welche ausgestaltet sind, um für eine längere Messdauer, beispielsweise einige Stunden, Tage oder Wochen, in der Probe zu verbleiben. Ein wesentliches Anwendungsbeispiel derartiger Sensoren sind implantierbare Sensoren, wie beispielsweise subkutane Sensoren, die unter die Haut eines Patienten (beispielsweise in das interstitielle Fettgewebe) implantiert werden können. Diese Sensoren sind in der Regel für eine kontinuierliche Überwachung der Analytkonzentration oder eine Überwachung der Analytkonzentration in kurzen Zeitabständen konzipiert und können beispielsweise über eine elektrische Verbindung Daten mit einem Messgerät austauschen. Beispiele derartiger implantierbarer Sensoren oder Testelemente, welche auf einem elektrochemischen Messprinzip beruhen, sind in EP 0 678 308 B1 oder in EP 0 748 183 B1 dargestellt. In beiden Fällen wird neben dem eigentlichen Aufbau des Sensors auch eine Einführhilfe beschrieben, mittels derer der Sensor implantiert werden kann. Die Einführhilfe kann nach dem Implantieren wieder aus dem Hautbereich entfernt werden.

Die zahlreichen Systemkomponenten (Lanzette, Stechhilfe, Testelement und Analysegerät) benötigen viel Platz und bedingen eine relativ komplexe Handhabung. Inzwischen gibt es auch Systeme mit einem höheren Grad der Integration und damit einer einfacheren Handhabung, bei denen z.B. die Testelemente im Analysegerät magaziniert und für die Messung zur Verfügung gestellt werden. Ein nächster Schritt der Miniaturisierung ist beispielsweise durch die Integration von mehreren Funktionen bzw. Funktionselementen in einem einzigen analytischen Hilfsmittel (Disposable) zu erreichen. Durch geeignete Kombination von Stechvorgang und sensorischer Analytkonzentrations-Erfassung auf einem Testelement lässt sich beispielsweise der Bedienablauf deutlich vereinfachen.

Im Stand der Technik ist es bekannt, die Lanzetten solcher analytischer Hilfsmittel mit einer Kapillarstruktur zu versehen oder sie zu einem Teil einer Kapillarstruktur zu machen (zum Beispiel aus WO 2005/104948 A1). Dabei wird die Lanzettenspitze nach dem Einstich und dem Probenaustritt an die Probe herangeführt, um diese mit der Kapillare zu sammeln.

Die Lanzettenspitze von zur Blutgewinnung verwendeten Lanzetten wird typischerweise im voraus sterilisiert und durch einen Sterilschutz (z. B. in Form einer Kappe oder Tasche) in einem sterilen Zustand gehalten, bevor sie für einen Stechvorgang verwendet wird, um sicherzustellen, dass die Lanzettenspitze durch die Umgebung nicht kontaminiert wird. Ferner werden häufig Vorkehrungen dafür getroffen, dass die Lanzettenspitze nach einem erfolgten Stechvorgang abgeschirmt wird (gegebenenfalls durch dieselbe Kappe oder Tasche), so dass unbeabsichtigte Verletzungen und damit einhergehende Infektionen durch an der Lanzettenspitze anhaftendes Blut vermieden werden.

Bei Einzellanzetten kann beispielsweise ein Sterilschutz hergestellt werden, indem die Spitze der Lanzette in einem Arbeitsgang mit der Erzeugung des Lanzettenkörpers mit Kunststoff umspritzt wird. Vor Gebrauch entfernt der Anwender dieses Teil manuell, meist beim Einsetzen in eine Stechhilfe. Im Fall von magazinierten Lanzetten sind ähnliche Sterilschutzeinrichtungen gebräuchlich, z. B. solche, bei denen die Lanzette nach hinten aus einem Sterilschutz herausgezogen wird, woraufhin der Sterilschutz per Federkraft aus dem Stichweg befördert wird. Hierfür sind relativ aufwändige Mechaniken erforderlich, insbesondere Federn, die in das Verbrauchsmaterial integriert sind.

In WO 01/66010 wird die Umständlichkeit dieser Mechanik umgangen, indem der Sterilschutz einfach durchstochen wird. WO 01/66010 bezieht sich auf eine Lanzette enthaltend eine Lanzettennadel mit einer Spitze und einen Lanzettenkörper, der die Lanzettennadel zumindest im Bereich der Spitze vollständig umgibt. Der Lanzettenkörper besteht zumindest im Bereich der Spitze der Lanzettennadel aus einem elastischen Material, in das die Spitze der Lanzettennadel eingebettet ist. Ferner wird eine Lanzette beschrieben, die eine Lanzettennadel mit einer Spitze und einen Hohlkörper, der zumindest die Spitze der Lanzettennadel umgibt, enthält. Die Lanzettennadel ist im Bereich ihrer Spitze im Hohlkörper beweglich und der Hohlkörper besteht zumindest teilweise aus einem elastischen Material, das von der Spitze der Lanzettennadel beim Stechvorgang durchstoßen werden kann und das sich gegebenenfalls nach dem Zurückziehen der Spitze der Lanzettennadel in den Hohlkörper wieder verschließt.

EP 1 360 935 A1 bezieht sich auf eine bandförmige Verpackung für eine Vielzahl von medizinischen Hilfsmitteln. Die Hilfsmittel sind in Vertiefungen eines ersten Bandabschnittes untergebracht, die mit einem zweiten Bandabschnitt bedeckt sind.

EP 1 492 457 B1 betrifft eine automatisch öffnende Verpackung für medizinische Hilfsmittel, die folgendes umfasst:
- einen oberen flexiblen Flächenabschnitt, der ein distales Ende, ein proximales Ende, einen ersten peripheren Rand und einen zweiten peripheren Rand aufweist,
- einen unteren flexiblen Flächenabschnitt, der ein distales Ende, ein proximales Ende, einen ersten peripheren Rand und einen zweiten peripheren Rand aufweist,
wobei der obere und der untere flexible Flächenabschnitt dazu geeignet sind, dass sie entlang zumindest eines Segments ihrer ersten und zweiten peripheren Ränder lösbar miteinander versiegelt werden können, wodurch ein medizinisches Gerät innerhalb des oberen und des unteren flexiblen Flächenabschnitts umschlossen wird. An dem distalen Ende des oberen flexiblen Flächenabschnitts und an dem distalen Ende des unteren flexiblen Flächenabschnitts ist ein Bund angebracht, wobei der Bund an den distalen Enden des unteren und oberen flexiblen Flächenabschnitts in einer Weise angebracht ist, dass eine relative, abgleitende Bewegung des Bundes und der proximalen Enden des oberen und des unteren flexiblen Flächenabschnitts, die einen Abstand dazwischen verringert, dazu führt, dass sich ein Auseinanderziehen des oberen und des unteren flexiblen Flächenabschnitts ergibt. Dadurch wird automatisch die Verpackung geöffnet und zumindest ein Abschnitt des medizinischen Geräts freigelegt. Diese automatisch öffnende Verpackung hat unter anderem den Nachteil, dass die Lanzettenspitze in dem Bereich der Verpackung angeordnet ist, der mit der Bewegung des Bundes zurückgezogen wird. Daher besteht die Gefahr, dass die Lanzettenspitze auf einen der flexiblen Flächenabschnitte oder die Naht zwischen den beiden flexiblen Flächenabschnitten trifft. Dabei kann sie einen der flexiblen Flächenabschnitte durchstechen und beim weiteren Öffnen der Verpackung durch das Auseinanderziehen der Flächenabschnitte verbogen werden. Ferner kann die Lanzettenspitze auf eine Naht treffen, deren mechanische Einwirkung oder Kleber die Oberflächeneigenschaften der Lanzettenspitze nachteilig verändern kann. Falls die Lanzettenspitze eine Kapillarstruktur aufweist, muss diese zum Beispiel hydrophil sein, damit der Bluttransport funktioniert. Durch den Kontakt der Lanzettenspitze mit der Naht der Verpackung ist ihre Hydrophilie gefährdet. WO 2004/075760 offenbart einen Gegenstand gemäße der Präambeln der Ansprüche 1 und 12. EP1508304 beschreibt ebenfalls ein medizinisches Hilfsmittel mit Lanzette und sensor.

Die Aufgabe der vorliegenden Erfindung ist es daher, die Nachteile des Standes der Technik zu vermeiden und insbesondere ein medizinisches Hilfsmittel mit einer Lanzette oder einen Sensor mit mindestens einem Testelement bereitzustellen, die eine sterile Verpackung der Lanzette bzw. des Testelements gewährleisten, deren Öffnungsmechanismus nicht die Funktionsfähigkeit der Lanzette bzw. des Testelements gefährdet.

Diese Aufgabe wird erfindungsgemäß gelöst durch ein medizinisches Hilfsmittel gemäß Anspruch 1 und einen Sensor gemäß Anspruch 12, wobei in beiden Fällen die gemeinsame Grundidee in einer geeigneten Ausgestaltung der Verpackung besteht.

Es wird ein medizinisches Hilfsmittel vorgeschlagen, enthaltend eine Lanzette mit einem proximalen und einem distalen Ende, wobei die Lanzette einen Lanzettenkörper und an dem proximalen Ende eine Lanzettenspitze aufweist. Die Lanzette ist zumindest teilweise von einer Verpackung umschlossen, die zumindest einen flexiblen Folienabschnitt umfasst, der ein distales Ende, ein proximales Ende, einen ersten seitlichen Rand und einen zweiten seitlichen Rand aufweist. Die von der Verpackung zumindest teilweise umschlossene Lanzette ist verschiebbar zwischen einem oberen und einem unteren Schutzabschnitt angeordnet, Der mindestens eine flexible Folienabschnitt ist entlang zumindest eines Segments seiner ersten und zweiten seitlichen Ränder lösbar mit einem weiteren flexiblen Folienabschnitt oder einem der Schutzabschnitte verbunden. Der mindestens eine flexible Folienabschnitt ist ferner im Bereich der Lanzettenspitze mit einem weiteren flexiblen Folienabschnitt oder mit einem der Schutzabschnitte über eine lösbare Verbindung verbunden, so dass die Lanzettenspitze durch die Verpackung umschlossen ist. Der mindestens eine flexible Folienabschnitt ist weiterhin in einem Abstand zu der lösbaren Verbindung mit einem der Schutzabschnitte über eine feste Verbindung verbunden. Die proximalen Enden des unteren und oberen Schutzabschnitts sind auseinander biegbar, wodurch der Abstand zwischen der lösbaren und der festen Verbindung vergrößert und dadurch eine Zugkraft auf die lösbare Verbindung ausgeübt wird, so dass ein Öffnen der Verpackung an der lösbaren Verbindung erfolgt und eine proximale Öffnung entsteht, ohne dass die Lanzettenspitze die Verpackung durchstechen muss. Durch die proximale Öffnung ist zumindest ein Teil der Lanzette in proximaler Richtung aus der Verpackung herausschiebbar.

Die Erfindung bezieht sich insbesondere auf ein medizinisches Hilfsmittel, enthaltend eine Lanzette mit einem proximalen und einem distalen Ende, wobei die Lanzette einen Lanzettenkörper und an dem proximalen Ende eine Lanzettenspitze aufweist und wobei die Lanzette zumindest teilweise von einer flexiblen Verpackung umschlossen ist. Die flexible Verpackung umfasst einen oberen flexiblen Folienabschnitt, der ein distales Ende, ein proximales Ende, einen ersten seitlichen Rand und einen zweiten seitlichen Rand aufweist. Die flexible Verpackung umfasst ferner einen unteren flexiblen Folienabschnitt, der ein distales Ende, ein proximales Ende, einen ersten seitlichen Rand und einen zweiten seitlichen Rand aufweist. Der obere und der untere flexible Folienabschnitt sind entlang zumindest eines Segments ihrer ersten und zweiten seitlichen Ränder lösbar miteinander verbunden und umschließen die Lanzette zumindest teilweise. Die von der flexiblen Verpackung zumindest teilweise umschlossene Lanzette ist verschiebbar zwischen einem oberen und einem unteren Schutzabschnitt angeordnet. Der untere flexible Folienabschnitt und der obere flexible Folienabschnitt sind über eine proximale Naht lösbar miteinander verbunden, wodurch die Lanzettenspitze durch die flexible Verpackung umschlossen ist. Eine Naht ist in diesem Zusammenhang eine Verbindungslinie der zwei miteinander verbundenen flexiblen Folienabschnitte, die durch eine beliebige, dem Fachmann bekannte Verbindungstechnik hergestellt werden kann, insbesondere durch Siegeln oder Kleben. Das proximale Ende des unteren flexiblen Folienabschnitts ist fest mit dem unteren Schutzabschnitt und das proximale Ende des oberen flexiblen Folienabschnitts ist fest mit dem oberen Schutzabschnitt verbunden. Die proximalen Enden des unteren und oberen Schutzabschnitts sind auseinanderbiegbar zum Öffnen der flexiblen Verpackung an der proximalen Naht, ohne dass die Lanzettenspitze die flexible Verpackung durchstechen muss. Daraufhin ist zumindest ein Teil der Lanzette aus der flexiblen Verpackung und den Schutzabschnitten in proximaler Richtung herausschiebbar.

Ein medizinisches Hilfsmittel ist in diesem Zusammenhang insbesondere eine Lanzettenvorrichtung oder eine Stechhilfe zur Probengewinnung durch Einstechen oder ein analytisches Hilfsmittel, das die Funktionen Einstechen und Probenaufnahme oder die Funktionen Einstechen, Probenaufnahme und Bereitstellen einer Testchemie zur Analyse der Probe zusammenfasst.

Im Zusammenhang mit der Erfindung ist das proximale Ende einer Komponente des analytischen Hilfsmittels jeweils das für den Einstich der Lanzettenspitze der Haut des Patienten zugewandte Ende der Komponente. Das distale Ende ist das dem proximalen Ende entgegengesetzte Ende der Komponente.

Die Lanzettenspitze der Lanzette des erfindungsgemäßen Hilfsmittels dient zum Perforieren der Haut eines Patienten zur Gewinnung einer Blutprobe oder einer Probe aus interstitieller Flüssigkeit. Die Lanzette ist zumindest teilweise von der (vorzugsweise flexiblen) Verpackung umschlossen, wobei die Verpackung zumindest die Lanzettenspitze umschließt, um diese im unbenutzten Zustand bis unmittelbar vor der Benutzung zu schützen und vorzugsweise steril (keimfrei) zu halten.

Die Verpackung umfasst zumindest einen flexiblen Folienabschnitt. Gemäß einer bevorzugten Ausführungsform der vorliegenden Erfindung handelt es sich um eine flexible Verpackung, die einen oberen und einen unteren flexiblen Folienabschnitt umfasst.

Flexibel bedeutet in diesem Zusammenhang, dass die Folienabschnitte faltbar und beim Öffnen der Verpackung so biegsam sind, dass sie sich durch geringe Krafteinwirkung von der Lanzettenspitze wegbewegen lassen. Für die flexiblen Folienabschnitte bzw. die flexible Verpackung wird vorzugsweise ein Material verwendet, das sich gut kleben lässt, das durch ein geeignetes Sterilisationsverfahren für die Lanzette nicht beschädigt wird (insbesondere nicht versprödet) und das keine Stoffe an die Lanzette abgibt, die nicht mit der Haut oder einer Öffnung in der Haut eines Patienten in Kontakt kommen sollten. Vorzugsweise handelt es sich bei dem Material der flexiblen Folienabschnitte bzw. der flexiblen Verpackung um ein Polymer, insbesondere PET, PP, oder Styrol, um Papier oder um ein Metall, insbesondere Aluminium, Edelstahl oder Zinn. Ein flexibler Folienabschnitt bzw. eine flexible Verpackung aus einer Polymerfolie hat bevorzugt eine Dicke im Bereich von 3 bis 100 µm, besonders bevorzugt von 5 bis 15 µm. Ein flexibler Folienabschnitt bzw. eine flexible Verpackung aus Papier wird vorzugsweise aus dem Papier hergestellt, wie es für die Blisterverpackung von Einwegspritzen verwendet wird. Ein flexibler Folienabschnitt bzw. eine solche flexible Verpackung aus Papier hat bevorzugt eine Dicke in einem Bereich von 50 bis 150 µm, besonders bevorzugt von ca. 100 µm. Für einen flexiblen Folienabschnitt bzw. eine flexible Verpackung aus Metall wird vorzugsweise ein Metall verwendet, das keine Ionen an die Lanzette abgibt. Ein flexibler Folienabschnitt bzw. eine flexible Verpackung aus Metall hat bevorzugt eine Dicke im Bereich von 30 bis 70 µm, besonders bevorzugt von 50 µm.

Die oberen und unteren flexiblen Folienabschnitte bilden bei der bevorzugten Ausführungsform mit flexibler Verpackung eine zumindest einen Teil der Lanzette umgebende Folientasche, da sie an ihren seitlichen Rändern, an der proximalen Naht und ihren distalen Enden miteinander verbunden sind. Die proximalen Enden der beiden flexiblen Folienabschnitte ragen über die proximale Naht hinaus und sind jeweils mit einem angrenzenden Schutzabschnitt fest verbunden.

Fest verbunden heißt im Zusammenhang mit der vorliegenden Erfindung, dass sich die feste Verbindung nicht durch die beim Öffnen der Verpackung und beim Herausschieben der Lanzette wirkenden Kräfte löst. Im Unterschied zu der festen Verbindung ist die lösbare Verbindung so gestaltet (z.B. geklebt, gesiegelt ...), dass sie sich beim Öffnen der Verpackung löst.

Die zwei Schutzabschnitte sind starrer als die flexiblen Folienabschnitte ausgebildet, damit sie beim Verschieben der Lanzette diese Relativbewegung zulassen, die proximalen Enden der flexiblen Folienabschnitte festhalten und vor und nach der Benutzung einen Schutz für die Lanzette, zum Beispiel gegen Druckausübung oder Knicken, gewährleisten. Die Lanzette ist zwischen dem oberen und dem unteren Schutzabschnitt angeordnet, so dass sie vor dem Öffnen der flexiblen Verpackung nicht über die proximalen Enden der Schutzabschnitte hinausragt.

Gemäß einer Ausführungsform der vorliegenden Erfindung umfasst die Verpackung nur einen flexiblen Folienabschnitt, der mit einem der Schutzabschnitte entlang seiner seitlichen Ränder verbunden ist. Die Lanzette ist dabei zwischen dem flexiblen Folienabschnitt und dem Schutzabschnitt angeordnet und wird durch diese zumindest teilweise umschlossen. Der flexible Folienabschnitt ist mit seinem proximalen Ende mit einem der beiden Schutzabschnitte (z.B. dem oberen) über eine lösbare Verbindung verbunden. Von dort erstreckt sich der flexible Folienabschnitt quer vor der Lanzettenspitze zu dem anderen der beiden Schutzabschnitte (z.B. dem unteren) hin, wo er im Bereich der Lanzettenspitze über eine feste Verbindung mit dem anderen Schutzabschnitt (z.B. dem unteren) verbunden ist. Der Rest des flexiblen Folienabschnitts flankiert die Lanzette und kann z.B. an seinem distalen Ende mit einem der beiden Schutzabschnitte (z.B. dem oberen) fest verbunden sein, um zumindest einen Teil der Lanzette zu umschließen. In diesem Fall wird also die Verpackung der Lanzette durch den flexiblen Folienabschnitt und einen der Schutzabschnitte gebildet.

Zum Öffnen der (ggf. flexiblen) Verpackung werden bei der vorliegenden Erfindung die proximalen Enden der Schutzabschnitte (vorzugsweise elastisch) auseinandergebogen. Das Auseinanderbiegen kann dabei z.B. durch das Verbiegen beider Schutzabschnitte in entgegengesetzten Richtungen oder durch das Wegbiegen eines der beiden Schutzabschnitte von dem anderen, während der andere starr in seiner Position verbleibt, erfolgen. Folglich ist vorzugsweise einer der beiden Schutzabschnitte zum Verbiegen seines proximalen Endes elastisch verformbar, während der andere entweder ebenfalls (zumindest beim proximalen Ende) elastisch verformbar oder starrer ausgebildet ist.

Bei der bereits beschriebenen bevorzugten Ausführungsform der vorliegenden Erfindung mit einer flexiblen Verpackung aus zwei flexiblen Folienabschnitten, die eine proximale Verbindungsnaht vor der Lanzettenspitze aufweisen, werden die proximalen Enden der flexiblen Folienabschnitte, die fest an den beiden Schutzabschnitten befestigt sind, durch die Vergrößerung des Abstandes zwischen den Schutzabschnitten beim Auseinanderbiegen ihrer proximalen Enden auseinandergezogen, so dass die lösbare Verbindung zwischen den flexiblen Folienabschnitten an der proximalen Naht durch die dabei wirkende Kraft gelöst wird. Es entsteht daher im Bereich der proximalen Naht eine proximale Öffnung in der flexiblen Verpackung. Durch diese proximale Öffnung kann die Lanzette (beginnend mit der Lanzettenspitze) aus der flexiblen Verpackung heraus geschoben werden, wobei sie sich in proximaler Richtung relativ zu den Schutzabschnitten bewegt, bis die Lanzettenspitze über die proximalen Enden der Schutzabschnitte hinausragt und einen Einstechvorgang zur Probengewinnung durchführen kann.

Bei der bereits beschriebenen Ausführungsform der vorliegenden Erfmdung mit nur einem flexiblen Folienabschnitt, der im Bereich der Lanzettenspitze mit einem Schutzabschnitt fest und mit dem anderen Schutzabschnitt lösbar verbunden ist, wird das proximale Ende des flexiblen Folienabschnitts durch die Vergrößerung des Abstandes zwischen den Schutzabschnitten und damit auch zwischen der festen und der lösbaren Verbindung beim Auseinaderbiegen der beiden Schutzabschnitte von dem Schutzabschnitt weg gezogen, so dass die lösbare Verbindung zwischen dem proximalen Ende des flexiblen Folienabschnitts und dem Schutzabschnitt durch die dabei wirkende Zugkraft gelöst wird. Es entsteht daher im Bereich der lösbaren Verbindung eine proximale Öffnung in der Verpackung. Durch diese proximale Öffnung kann die Lanzette (beginnend mit der Lanzettenspitze) aus der Verpackung herausgeschoben werden, wobei sie sich in proximaler Richtung relativ zu den Schutzabschnitten bewegt, bis die Lanzettenspitze über die proximalen Enden der Schutzabschnitte hinausragt und einen Einstechvorgang zur Probengewinnung durchführen kann.

Die proximale Öffnung in der Verpackung wird erfindungsgemäß ausschließlich durch das Auseinanderbiegen der beiden Schutzabschnitte erreicht, ohne dass die Lanzettenspitze für das Öffnen der (bevorzugt flexiblen) Verpackung benutzt wird, insbesondere ohne dass die Lanzettenspitze die Verpackung durchsticht.

Beim Schieben der Lanzette durch die proximale Öffnung aus der (bevorzugt flexiblen) Verpackung heraus öffnet sich die Verpackung gegebenenfalls automatisch weiter, insbesondere an den seitlichen Rändern des mindestens einen flexiblen Folienabschnitts, da der mindestens eine flexible Folienabschnitte weiterhin über die feste(n) Verbindung(en) mit den Schutzabschnitten verbunden bleibt, die Lanzette sich relativ zu den Schutzabschnitten und damit auch relativ zu der festen Verbindung des mindestens einen flexiblen Folienabschnitts bewegt und sich außerdem ausgehend von der Lanzettenspitze zum Lanzettenkörper hin verbreitert.

Die distalen Enden des mindestens einen flexiblen Folienabschnitts sind vorzugsweise fest mit dem distalen Ende der Lanzette verbunden, so dass sie sich mit der Lanzette mitbewegen und ein "Herausschälen" der Lanzette aus der flexiblen Verpackung beim Verschieben der Lanzette relativ zu den Schutzabschnitten und relativ zu den damit fest verbundenen Bereichen der Folienabschnitte verursachen.

Nach der Benutzung kann die Lanzettenspitze wieder vollständig zwischen die zwei Schutzabschnitte zurückgezogen werden, so dass der Benutzer vor einem unbeabsichtigten weiteren Stechvorgang geschützt ist. Die zurückgezogene Lanzette wird vorzugsweise wieder durch die (bevorzugt flexible) Verpackung beidseitig bedeckt.

Das erfindungsgemäße medizinische Hilfsmittel hat unter anderem den Vorteil, dass sich die (bevorzugt flexible) Verpackung, die vorzugsweise als Sterilschutz für die Lanzette dient, weitgehend automatisch öffnet, ohne die Lanzettenspitze zu beschädigen und ohne eine Gefahr eines unbeabsichtigten Stechens mit der Lanzette für den Benutzer. Das Öffnen des Sterilschutzes erfordert keine umständliche oder komplizierte Handhabung des medizinischen Hilfsmittels. Die einfache Gestaltung des medizinischen Hilfsmittels ermöglicht ferner eine kostengünstige Produktion, insbesondere eine kostengünstige Massenproduktion. Die Lanzette ist vor der Benutzung durch die Schutzabschnitte vor einer Beschädigung und durch die Verpackung vor einer Kontamination geschützt. Die Schutzabschnitte schützen ferner den Benutzer vor einem unbeabsichtigten Kontakt mit der Lanzettenspitze.

Die Erfindung bezieht sich weiterhin auf ein Verfahren zum Entpacken eines erfindungsgemäßen medizinischen Hilfsmittels mit den Schritten:
- Auseinanderbiegen der proximalen Enden des unteren und oberen Schutzabschnitts, wodurch die Verpackung an der lösbaren Verbindung geöffnet wird, so dass eine proximale Öffnung entsteht und
- Herausschieben zumindest eines Teils der Lanzette aus der (bevorzugt) flexiblen Verpackung durch die proximale Öffnung und aus den Schutzabschnitten in proximaler Richtung, so dass zumindest die Lanzettenspitze über die proximalen Enden der Schutzabschnitte hinausragt.

Die Vorteile und Details zum Ablauf dieses Verfahrens wurden bereits im Zusammenhang mit dem erfindungsgemäßen medizinischen Hilfsmittel erläutert. Insbesondere kann das Auseinanderbiegen dabei z.B. durch das Verbiegen beider Schutzabschnitte in entgegengesetzten Richtungen oder durch das Wegbiegen eines der beiden Schutzabschnitte von dem anderen, während der andere starr in seiner Position verbleibt, erfolgen.

Eine Lanzette ist im Zusammenhang mit der vorliegenden Erfindung ein Stechinstrument, das zum Erzeugen einer Perforation in der Haut eines Patienten geeignet ist. Dabei kann es sich z.B. um einen massiven nadelförmigen Körper handeln. Ein weiteres Beispiel für eine Lanzette im Sinne der vorliegenden Erfindung ist die Nadel des subkutanen Glukosesensor-Sets, das aus der US 6,520,326 B2 bekannt ist.

Gemäß einer bevorzugten Ausführungsform der vorliegenden Erfindung weist die Lanzette eine durch einen halboffenen Kanal gebildete Kapillarstruktur auf. Die Kapillarstruktur dient zur Aufnahme einer Probe nach dem Stechvorgang, wobei die Probe durch Kapillarkräfte in die hydrophil ausgebildete Kapillarstruktur aufgenommen werden kann. Durch das Öffnen der (bevorzugt flexiblen) Verpackung ohne eine Einwirkung der Lanzettenspitze wird eine Beschädigung, Verschmutzung oder Beeinträchtigung von Oberflächeneigenschaften der Kapillarstruktur vermieden.

Gemäß einer bevorzugten Ausführungsform der vorliegenden Erfindung wirkt das medizinische Hilfsmittel mit einem durch mindestens einen der Schutzabschnitte hindurchgreifenden Mitnehmer zusammen, der mit dem Lanzettenkörper verbunden wird und gemeinsam mit der Lanzette relativ zu den Schutzabschnitten in proximaler Richtung verschiebbar ist. Gemäß einer weiteren bevorzugten Ausführungsform der vorliegenden Erfindung enthält das medizinische Hilfsmittel einen durch mindestens einen der Schutzabschnitte hindurchgreifenden Mitnehmer, der mit dem Lanzettenkörper verbunden und gemeinsam mit der Lanzette relativ zu den Schutzabschnitten in proximaler Richtung verschiebbar ist. Der Mitnehmer dient in beiden Fällen zum zumindest teilweisen Herausschieben der Lanzette aus der (bevorzugt flexiblen) Verpackung und den Schutzabschnitten automatisch durch eine dazu vorgesehene Komponente eines Analysesystems oder einer Stechhilfe oder manuell durch einen Benutzer. Dazu können (z.B. durch den Benutzer) entweder die Schutzabschnitte festgehalten und der Mitnehmer relativ zu den Schutzabschnitten verschoben oder der Mitnehmer festhalten und die Schutzabschnitte relativ zu dem Mitnehmer verschieben. Vorzugsweise ist dazu zumindest in einem der Schutzabschnitte eine Ausnehmung vorgesehen, durch die der Mitnehmer beim Verschieben gleitet und geführt wird.

Gemäß einer bevorzugten Ausführungsform der vorliegenden Erfindung wird die Lanzette automatisch zwischen den Schutzabschnitten verschoben. Dazu weist das medizinische Hilfsmittel oder eine das medizinische Hilfsmittel aufnehmende Stechhilfe z.B. einen angetriebenen Stößel auf, der zum Verschieben direkt auf das distale Ende der Lanzette oder über einen Mitnehmer an einer sonstigen Position auf den Lanzettenkörper in proximaler Richtung einwirkt.

Bei dem erfindungsgemäßen medizinischen Hilfsmittel in der Ausführungsform mit einem oberen und einem unteren flexiblen Folienabschnitt kann der Lanzettenkörper am distalen Ende der flexiblen Verpackung in die flexible Verpackung eingeklebt sein. Bei dem erfindungsgemäßen medizinischen Hilfsmittel in der Ausführungsform mit nur einem flexiblen Folienabschnitt kann der Lanzettenkörper am distalen Ende zwischen dem flexiblen Folienabschnitt und einem der Schutzabschnitte eingeklebt sein.

Vorzugsweise sind die oberen und unteren flexiblen Folienabschnitte der bereits beschriebenen Ausführungsform der Erfindung mit flexibler Verpackung Abschnitte einer einzigen flexiblen Folie, die an den distalen Enden der flexiblen Folienabschnitte umgebogen ist. Dabei ist die flexible Folie zum Beispiel um das distale Ende des Lanzettenkörpers gefaltet, so dass sich ein oberer und ein unterer flexibler Folienabschnitt ergeben. Diese beiden flexiblen Folienabschnitte können dann entlang ihrer seitlichen Ränder und quer vor der Lanzettenspitze lösbar miteinander verbunden werden, zum Beispiel durch Versiegeln oder Verkleben, um die Lanzette vollständig als flexible Verpackung zu umschließen.

Die (bevorzugt flexible) Verpackung kann bei der vorliegenden Erfindung einen Teil der Lanzette, die gesamte Lanzette oder weitere Komponenten des medizinischen Hilfsmittels (zum Beispiel Teile eines Mitnehmers) zusätzlich zu einem Teil der Lanzette oder der gesamten Lanzette umschließen oder bedecken.

Gemäß einer bevorzugten Ausführungsform der vorliegenden Erfindung trägt bei dem erfindungsgemäßen medizinischen Hilfsmittel das proximale Ende einer der Schutzabschnitte innen ein Testfeld, das zur Analyse einer Probe vorgesehen ist. Dazu kann zumindest einer der Schutzabschnitte gegenüber dem mindestens einen flexiblen Folienabschnitt bzw. der flexiblen Verpackung in proximaler Richtung ausreichend verlängert sein, um das Testfeld auf seiner Innenfläche in dem verlängerten Bereich zu enthalten. Zur Analyse wird eine Probe in das Testfeld transportiert, wo sie gegebenenfalls mit einer oder mehreren Reagenzien reagiert, bevor sie analysiert wird. Die Analyse kann zum Beispiel optisch, insbesondere photometrisch, oder elektrochemisch durchgeführt werden. Zur photometrischen Analyse enthalten die Testelemente ein Reagenzsystem, dessen Reaktion mit dem Analyten zu einer photometrisch nachweisbaren Veränderung (einem Farbumschlag) führt. Die Reagenzien befinden sich dabei üblicherweise in einem Testfeld des Testelements, wobei deren Farbe sich in Abhängigkeit von der Konzentration ändert. Diese Farbänderung lässt sich mit Hilfe einer Messanordnung quantitativ durch Reflexionsphotometrie bestimmen.

Elektrochemische Testelemente enthalten ein elektrochemisches Reagenzsystem, dessen Reaktion mit dem Analyten die zwischen zwei Polen des Testelements anliegende elektrische Spannung und/oder die zwischen zwei Polen des Testelements bei definierter Spannung fließende Stromstärke beeinflusst. In diesem Fall ist also die Spannung oder Stromstärke die physikalisch messbare Größe, die mit einer entsprechenden in dem Analysesystem integrierten, als Spannungs- oder Strommesseinrichtung gestalteten Messanordnung bestimmt und deren mit der Konzentration des Analyten korrelierende Änderung in die Analysedaten (Konzentration des Analyten) umgerechnet wird.

Bei dem erfindungsgemäßen medizinischen Hilfsmittel kann zum Beispiel der das Testfeld tragende Schutzabschnitt zumindest im Bereich des Testfeldes transparent gestaltet sein, so dass ein dafür vorgesehenes Analysesystem eine photometrische Analyse einer Probe auf dem Testfeld durchführen kann, wobei Licht von einer Lichtquelle durch den transparenten Bereich auf das Testfeld und daran reflektiert erneut durch den transparenten Bereich zu einem Detektor gelangen kann.

Vorzugsweise wird die Probe bei dieser Ausführungsform des erfindungsgemäßen medizinischen Hilfsmittels auf das innen am proximalen Ende einer der Schutzabschnitte angeordnete Testfeld durch Aufnehmen der Probe in eine durch einen halboffenen Kanal gebildete Kapillarstruktur der Lanzette und Abstreifen der Probe auf das Testfeld aufgenommen. Dazu kann das Testfeld zum Beispiel durch einen Benutzer aktiv gegen die Kapillarstruktur mit der darin enthaltenen Probe gedrückt werden, nachdem die Lanzette in die entsprechende Position zurückgezogen wurde.

Es kann aber auch eine Kapillare an oder in dem Schutzabschnitt, der das Testfeld trägt, vorgesehen sein, die die Probe mittels Kapillarkräften von einem Probenaufgabeort auf dem Schutzabschnitt zu dem Testfeld transportiert. Ferner ist eine direkte Aufgabe der Probe auf ein Testfeld möglich, falls das Testfeld in einer exponierten Position auf dem Schutzabschnitt angeordnet ist.

Gemäß einer Ausführungsform der vorliegenden Erfindung sind die distalen Enden der Schutzabschnitte mit einem Halteelement verbunden, das ein Distanzstück enthält, mittels dessen die distalen Enden der Schutzabschnitte in einem definierten Abstand zueinander gehalten werden. Vorzugsweise sind die Schutzabschnitte bei der vorliegenden Erfindung zwei rechteckige Abschnitte (zum Beispiel aus Folie oder Pappe), die (vorzugsweise in Ruheposition vor der Benutzung parallel zueinander) in einem definierten Abstand ihrer distalen Enden angeordnet sind und zumindest an ihren proximalen Enden eine entsprechend breite Öffnung aufweisen, durch die die Lanzette zumindest teilweise nach außen geschoben werden kann. Damit die Schutzabschnitte in dem festen Abstand gehalten werden können, ist beispielsweise ein Halteelement mit einem Distanzstück vorgesehen. An dem Halteelement kann ein Benutzer ferner das medizinische Hilfsmittel während der Benutzung festhalten.

Wie oben dargelegt, lässt sich die erfindungsgemäße Grundidee der Bereitstellung einer Sterilverpackung, welche die Nachteile der aus dem Stand der Technik bekannten Sterilverpackungen vermeidet, auch auf andere medizinische oder chemische Vorrichtungen anwenden. Insbesondere wird vorgeschlagen, diese Grundidee auf einen Sensor zum Nachweis mindestens eines Analyten in einer Probe anzuwenden. Bei dem mindestens einen Analyten kann es sich beispielsweise um Glukose oder einen anderen Analyten, insbesondere einen Metaboliten, handeln, wie beispielsweise Cholesterin, Laktat oder andere Arten von Analyten. Insbesondere kann es sich bei der Probe um eine flüssige Probe handeln, insbesondere eine Körperflüssigkeit wie Blut oder interstitielle Flüssigkeit.

Der vorgeschlagene Sensor umfasst mindestens ein Testelement, welches für den Nachweis des mindestens einen Analyten (wobei ein qualitativer und/oder quantitativer Nachweis möglich sein kann) geeignet ist. Dieses Testelement kann beispielsweise einem der eingangs beschriebenen, aus dem Stand der Technik bekannten Testelemente entsprechen und kann beispielsweise einen elektrochemischen und/oder optischen Nachweis des Analyten durchführen. So kann das Testelement beispielsweise streifenförmig, röhrchenförmig oder auf andere Art ausgestaltet sein. Der Sensor kann ein oder mehrere derartiger Testelemente umfassen und kann beispielsweise eine reine Aufbewahrungs- oder Magazinierungsfunktion des bzw. der Testelemente übernehmen oder kann, alternativ oder zusätzlich, auch eine Ansteuer- und Auswerteelektronik und/oder eine Optik enthalten. Insbesondere kann für die Ausgestaltung der Sensoren auf die in EP 0 678 308 B1 und EP 0 748 183 B1 dargestellten implantierbaren Sensoren verwiesen werden, wobei die nachfolgend beschriebene Verpackung nicht nur den eigentlichen flexiblen Fühler (welcher dem Testelement entspricht) ganz oder teilweise umschließen kann, sondern auch beispielsweise Bestandteile der Einführeinheit, beispielsweise die dort dargestellte hohle Einführnadel. So ist es insbesondere bevorzugt, wenn diejenigen Teile des Testelements und/oder die Teile des Sensors, welche direkt mit Körperflüssigkeit oder Körpergewebe in Kontakt geraten, also Teile, welche zumindest vorübergehend implantiert werden, zumindest teilweise von der Verpackung umschlossen sind. Das proximale Ende des Testelements sollte somit die implantierten Teile umfassen. Andere Bestandteile, insbesondere Bestandteile, welche außerhalb des Körpergewebes verbleiben, können auch außerhalb der Verpackung verbleiben. Insbesondere kann das Testelement beispielsweise eine Testchemie umfassen, welcher durch eine Kapillarstruktur oder eine ähnliche fluidische Struktur Flüssigkeit zugeführt wird, wobei die Testchemie nicht in den Körper implantiert wird. In diesem Fall kann die Testchemie auch außerhalb der Verpackung verbleiben. Auch eine Anwendung auf andere Arten von Sensorelementen, beispielsweise auf Sensorelemente mit bandförmigen Testelementen, wie beispielsweise in EP 0 299 517 B1 dargestellt, ist möglich.

Das Testelement umfasst ein proximales und ein distales Ende, analog zur Ausgestaltung der Lanzette des oben dargestellten medizinischen Hilfsmittels, wobei jedoch grundsätzlich jeder Bereich des Testelements als proximales oder distales Ende definiert werden kann.

Vorzugsweise sind das proximale und das distale Ende des Testelements auf einander gegenüberliegenden Seiten des Testelements angeordnet. Das Testelement kann insbesondere eine aktive Fläche umfassen, welche dem Nachweis des mindestens einen Analyten dient. Beispielsweise kann diese aktive Fläche ein oder mehrere Testfelder umfassen, welche beispielsweise eine Nachweischemie zum Nachweis des mindestens einen Analyten aufweisen. Wie oben beschrieben, kann dieser Nachweis beispielsweise auf elektrochemische und/oder optische Weise erfolgen. Vorzugsweise ist die aktive Fläche des mindestens einen Testelements im Bereich des proximalen Endes des Testelements angeordnet. Allerdings kann die aktive Fläche des Testelements auch ganz oder teilweise außerhalb der Verpackung verbleiben, insbesondere wenn, wie oben beschrieben, dieser aktiven Fläche Probenflüssigkeit zugeführt wird. Das proximale Ende des Testelements (und damit der verpackte Teil des Testelements) sollte jedoch die zu implantierenden Teile des Testelements und/oder anderer Bestandteile des Sensors umfassen welche implantiert werden.

Das Testelement ist, analog zu der Lanzettenspitze des medizinischen Hilfsmittels, zumindest teilweise von einer Verpackung umschlossen. Wie oben dargelegt, können dabei ein oder mehrere Testelemente in der Verpackung aufgenommen sein. Neben dem eigentlichen Testelement, welches dem Nachweis des mindestens einen Analyten dient, können auch, wie oben beschrieben, weitere Elemente in der Verpackung aufgenommen sein, beispielsweise Bestandteile einer Einführhilfe für ein implantierbares Testelement, insbesondere Bestandteile, welche zumindest vorübergehend implantiert werden.

Bezüglich der Ausgestaltung der Verpackung, welche die Grundidee der Erfindung beinhaltet, kann weitgehend auf die obige Beschreibung verwiesen werden. Sämtliche im Zusammenhang mit dem medizinischen Hilfsmittel beschriebenen Ausgestaltungen der Verpackung sind analog auch im Rahmen des vorgeschlagenen Sensors realisierbar.

So umfasst die Verpackung mindestens einen flexiblen Folienabschnitt, der ein distales Ende, ein proximales Ende, einen ersten seitlichen Rand und zweiten seitlichen Rand aufweist. Weiterhin ist das von der Verpackung zumindest teilweise umschlossene Testelement verschiebbar zwischen einem oberen und einem unteren Schutzabschnitt angeordnet, wobei der mindestens eine flexible Folienabschnitt entlang zumindest eines Segments seiner ersten und zweiten seitlichen Ränder lösbar mit einem weiteren flexiblen Folienabschnitt oder einem der Schutzabschnitte verbunden ist. Weiterhin ist der mindestens eine flexible Folienabschnitt im Bereich des proximalen Endes des Testelements mit einem weiteren flexiblen Folienabschnitt oder mit einem der Schutzabschnitte über eine lösbare Verbindung verbunden, so dass das proximale Ende des Testelements durch die Verpackung umschlossen ist. Der mindestens eine flexible Folienabschnitt ist in einem Abstand zu der lösbaren Verbindung mit einem der Schutzabschnitte über eine feste Verbindung verbunden. Die proximalen Enden des unteren und oberen Schutzabschnitts sind auseinander biegbar, wodurch der Abstand zwischen der lösbaren und der festen Verbindung vergrößerbar und dadurch eine Zugkraft auf die lösbare Verbindung ausübbar ist, so dass ein Öffnen der Verpackung an der lösbaren Verbindung erfolgt und eine proximale Öffnung entsteht, durch die zumindest ein Teil des Testelements in proximaler Richtung aus der Verpackung herausschiebbar ist.

Für die weiteren möglichen Ausgestaltungen des Sensors kann auf die obige Beschreibung der möglichen Ausgestaltungen des medizinischen Hilfsmittels verwiesen werden. Dabei ist sinngemäß die Lanzette jeweils durch das Testelement zu ersetzen, und die Lanzettenspitze jeweils durch das proximale Ende des Testelements. Die dargestellten Vorteile der Verpackung sind analog auch auf die Vorteile der Verpackung des Sensors übertragbar.

Anhand der Zeichnung wird die Erfindung nachstehend näher erläutert.

Es zeigt:
- Figur 1: schematisch ein erfindungsgemäßes medizinisches Hilfsmittel im Schnitt vor der Benutzung mit ungeöffneter flexibler Verpackung,
- Figur 2: schematisch ein erfindungsgemäßes medizinisches Hilfsmittel gemäß Figur 1 im Schnitt nach dem Öffnen der flexiblen Verpackung und
- Figur 3: schematisch eine Draufsicht auf ein erfindungsgemäßes medizinisches Hilfsmittel mit ungeöffneter flexibler Verpackung.

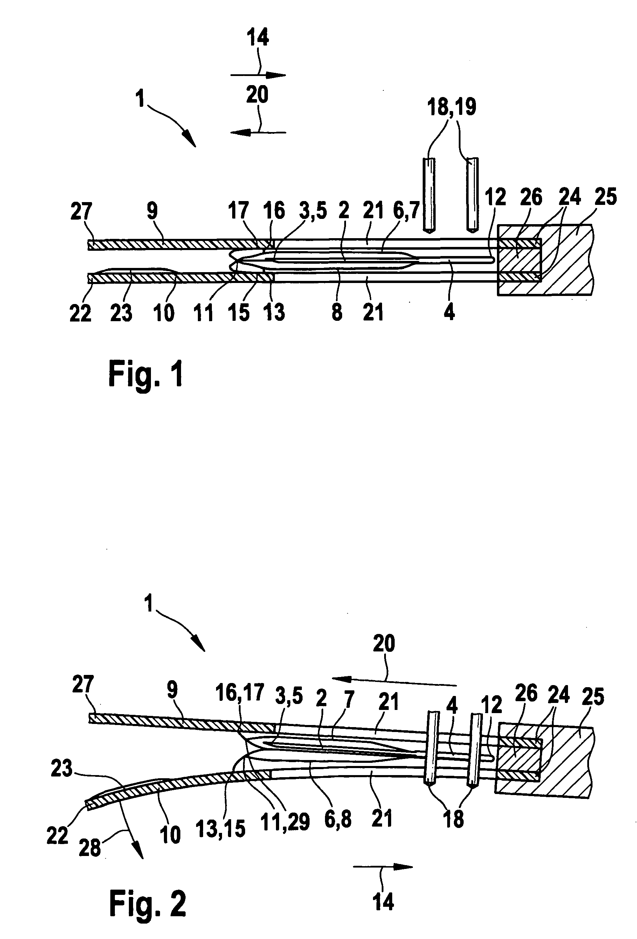

Figur 1 zeigt schematisch eine bevorzugte Ausführungsform eines erfindungsgemäßen medizinischen Hilfsmittels vor dem Öffnen der flexiblen Verpackung.

Das medizinische Hilfsmittel 1 enthält eine Lanzette 2 mit einer Lanzettenspitze 3 und einem Lanzettenkörper 4. Die Lanzettenspitze 3 ist am proximalen Ende 5 der Lanzette 2 angeordnet. Die Lanzette 2 ist von einer flexiblen Verpackung 6 umschlossen. Die flexible Verpackung 6 weist einen oberen flexiblen Folienabschnitt 7 und einen unteren flexiblen Folienabschnitt 8 auf. Diese flexiblen Folienabschnitte 7, 8 sind entlang ihrer (nicht dargestellten) seitlichen Ränder lösbar miteinander verbunden. Vor der Lanzettenspitze 3 sind die flexiblen Folienabschnitte 7, 8 über eine proximale Naht 11 lösbar miteinander verbunden. Die oberen und unteren flexiblen Folienabschnitte 7, 8 sind Abschnitte einer einzigen flexiblen Folie, die an den distalen Enden 12 der flexiblen Folienabschnitte 7, 8 um das distale Ende des Lanzettenkörpers 4 umgebogen ist. Der Lanzettenkörper 4 ist dabei am distalen Ende in die flexible Verpackung 6 eingeklebt. Die Lanzette 2 ist verschiebbar zwischen einem oberen Schutzabschnitt 9 und einem unteren Schutzabschnitt 10 angeordnet. Das proximale Ende 13 des unteren flexiblen Folienabschnitts 8 ist an dem ersten Verbindungsort 15 fest mit dem unteren Schutzabschnitt 10 verbunden, wobei das proximale Ende 13 des unteren flexiblen Folienabschnitts 8 gegenüber der proximalen Naht 11 in distaler Richtung 14 zum ersten Verbindungsort 15 hin zurückgebogen ist. Das proximale Ende 16 des oberen flexiblen Folienabschnitts 7 ist an dem zweiten Verbindungsort 17 mit dem oberen Schutzabschnitt 9 verbunden, wobei das proximale Ende 16 des oberen flexiblen Folienabschnitts 7 gegenüber der proximalen Naht 11 in distaler Richtung 14 zum zweiten Verbindungsort 17 hin zurückgebogen ist.

Die Lanzette 2 weist eine (nicht dargestellte) durch einen halboffenen Kanal gebildete Kapillarstruktur auf, die eine Probe mittels Kapillarkräften aufnehmen kann.

Als Mitnehmer 18 sind bei der dargestellten Ausführungsform zwei Stifte 19 vorgesehen, die in zwei Löcher im Lanzettenkörper 4 eingreifen können, um die Lanzette 2 in proximaler Richtung 20 verschieben zu können. Der Mitnehmer 18 kann erst kurz vor dem Verschieben der Lanzette 2 mit der Lanzette 2 verbunden werden. Vorzugsweise wird er durch eine Komponente eines (nicht dargestellten) dafür vorgesehenen Antriebssystems angetrieben, um automatisch mit dem Mitnehmer 18 die Lanzette 2 zunächst in proximaler Richtung 20 und nach der Benutzung zurück in distaler Richtung 14 zu verschieben. In dem oberen und dem unteren Schutzabschnitt 9, 10 sind schlitzförmige Ausnehmungen 21 vorgesehen, durch die der Mitnehmer 18 beim Verschieben gleitet und geführt wird.

Die beiden Schutzabschnitte 9, 10 sind über die flexible Verpackung 6 hinaus in proximaler Richtung 20 verlängert. Das proximale Ende 22 des unteren Schutzabschnitts 8 trägt innen ein Testfeld 23, das zur Analyse einer Probe vorgesehen ist.

Die distalen Enden 24 der Schutzabschnitte 9, 10 sind mit einem Halteelement 25 verbunden, das ein Distanzstück 26 enthält, mittels dessen die Schutzabschnitte 9, 10 in einem definierten Abstand zueinander gehalten werden. Das Distanzstück 26 kann zum Beispiel aus einem Stück dicker Kunststofffolie gefertigt und zwischen den distalen Enden 24 der Schutzabschnitte 9, 10 befestigt werden, bevor diese durch das Halteelement 25 aufgenommen werden.

Figur 2 zeigt das medizinische Hilfsmittel aus Figur 1 und dient zur Erläuterung der Verwendung des medizinischen Hilfsmittels.

Zum Entpacken des medizinischen Hilfsmittels 1 werden die proximalen Enden 22, 27 der Schutzabschnitte 9, 10 auseinandergebogen, zum Beispiel durch Verbiegen des proximalen Endes 22 des unteren Schutzabschnitts 10 in der Biegerichtung 28. Dadurch werden die proximalen Enden 13, 16 der flexiblen Folienabschnitte 8, 7 aufgrund ihrer festen Verbindung mit den Schutzabschnitten 10, 9 an den Verbindungsorten 15, 17 auseinandergezogen, so dass sich die lösbare Verbindung an der proximalen Naht 11 löst, wodurch eine proximale Öffnung 29 entsteht.

Mittels des Mitnehmers 18 kann die Lanzette 2 dann in proximaler Richtung 20 durch die proximale Öffnung 29 und (ohne das Testfeld 23 zu zerkratzen) aus den Schutzabschnitten 9, 10 herausgeschoben werden, so dass zumindest die Lanzettenspitze 3 über die proximalen Enden 22, 27 der Schutzabschnitte 10, 9 hinausragt. In dieser Position kann die Lanzettenspitze in die Haut eines Benutzers eingestochen werden und anschließend die dadurch gewonnene Probe (Blut oder interstitielle Flüssigkeit) in die Kapillarstruktur der Lanzette 2 aufnehmen. Durch Herausschieben der Lanzette 2 aus der flexiblen Verpackung 6 wird diese gegebenenfalls noch weiter geöffnet, zum Beispiel entlang der seitlichen Ränder der flexiblen Folienabschnitte 7, 8.

Nach dem Einstich und der Probensammelphase wird die mit der Probe gefiillte Kapillarstruktur der Lanzette 2 mit Hilfe des Mitnehmers 18 bis auf die Höhe des Testfeldes 26 in distaler Richtung 14 zurückgezogen. Dann wird die Probe von der Lanzette 2 auf das Testfeld 23 abgestreift, wobei das Testfeld 23 durch das Zurückbiegen des proximalen Endes 22 des unteren Schutzabschnitts 10 entgegen der Biegerichtung 28 mit der Lanzette 2 zu einem definierten Zeitpunkt in Kontakt gebracht wird.

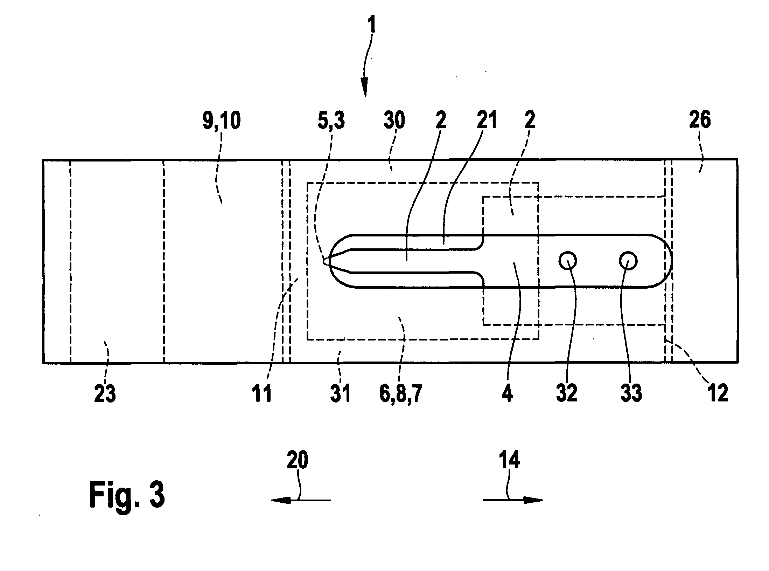

Figur 3 zeigt schematisch eine Draufsicht auf ein eine bevorzugte Ausführungsform eines erfindungsgemäßen medizinischen Hilfsmittels vor dem Öffnen der flexiblen Verpackung.

Das medizinische Hilfsmittel 1 enthält eine Lanzette 2 mit einer Lanzettenspitze 3 und einem Lanzettenkörper 4. Die Lanzettenspitze 3 ist am proximalen Ende 5 der Lanzette 2 angeordnet. Die Lanzette 2 ist von einer flexiblen Verpackung 6 umschlossen. Die flexible Verpackung 6 weist einen oberen flexiblen Folienabschnitt 7 und einen unteren flexiblen Folienabschnitt 8 auf. Diese flexiblen Folienabschnitte 7, 8 sind entlang ihrer seitlichen Ränder 30, 31 lösbar miteinander verbunden. Vor der Lanzettenspitze 3 sind die flexiblen Folienabschnitte 7, 8 über eine proximale Naht 11 lösbar miteinander verbunden. Die oberen und unteren flexiblen Folienabschnitte 7, 8 sind Abschnitte einer einzigen flexiblen Folie, die an den distalen Enden 12 der flexiblen Folienabschnitte 7, 8 um das distale Ende des Lanzettenkörpers 4 umgebogen ist. Der Lanzettenkörper 4 ist dabei am distalen Ende in die flexible Verpackung 6 eingeklebt. Die Lanzette 2 ist verschiebbar zwischen einem oberen Schutzabschnitt 9 und einem unteren Schutzabschnitt 10 angeordnet. Das proximale Ende des unteren flexiblen Folienabschnitts 8 ist an dem ersten Verbindungsort fest mit dem unteren Schutzabschnitt 10 verbunden. Das proximale Ende 16 des oberen flexiblen Folienabschnitts 7 ist an dem zweiten Verbindungsort fest mit dem oberen Schutzabschnitt 9 verbunden.

Die Lanzette 2 weist eine (nicht dargestellte) durch einen halboffenen Kanal gebildete Kapillarstruktur auf, die eine Probe mittels Kapillarkräften aufnehmen kann.

Als Mitnehmer 18 sind bei der dargestellten Ausführungsform zwei (nicht dargestellte) Stifte vorgesehen, die in zwei Löcher 32, 33 im Lanzettenkörper 4 eingreifen können, um die Lanzette 2 in proximaler Richtung 20 verschieben zu können. Der Mitnehmer 18 wird erst kurz vor dem Verschieben der Lanzette 2 mit der Lanzette 2 verbunden. In dem oberen und dem unteren Schutzabschnitt 9, 10 sind schlitzförmige Ausnehmungen 21 vorgesehen, durch die der Mitnehmer 18 beim Verschieben gleitet und geführt wird.

Die beiden Schutzabschnitte 9, 10 sind über die flexible Verpackung 6 hinaus in proximaler Richtung 20 verlängert. Das proximale Ende 22 des unteren Schutzabschnitts 8 trägt innen ein Testfeld 23, das zur Analyse einer Probe vorgesehen ist.

Die distalen Enden 24 der Schutzabschnitte 9, 10 sind über das Distanzstück 26 mit einem (nicht dargestellten) Halteelement verbunden. Das Distanzstück 26 hält die Schutzabschnitte 9, 10 in einem definierten Abstand zueinander. Das Distanzstück 26 kann zum Beispiel aus einem Stück dicker Kunststofffolie gefertigt und zwischen den distalen Enden 24 der Schutzabschnitte 9, 10 befestigt werden, bevor diese durch das Halteelement 25 aufgenommen werden.

Die obigen Ausführungsbeispiele, welche in den Figuren 1 bis 3 dargestellt sind, zeigen exemplarisch ein erfindungsgemäßes medizinisches Hilfsmittel 1. Es sei darauf hingewiesen, dass diese Ausführungsbeispiele analog auch auf einen erfindungsgemäßen Sensor übertragbar sind. Ausführungsbeispiele mit einem Sensor anstelle eines medizinischen Hilfsmittels sind daher zur Vereinfachung nicht dargestellt. Es wäre in diesen Ausführungsbeispielen jeweils die Lanzette 2 in den Figuren 1 bis 3 analog durch ein Testelement, beispielsweise eines der im Stand der Technik genannten Testelemente, zu ersetzen, und die Lanzettenspitze 3 würde dem proximalen Ende des Testelements entsprechen. Ansonsten sind die Ausführungsbeispiele analog übertragbar.

### Bezugszeichenliste

- 1: medizinisches Hilfsmittel
- 2: Lanzette
- 3: Lanzettenspitze
- 4: Lanzettenkörper
- 5: proximales Ende der Lanzette
- 6: flexible Verpackung
- 7: oberer flexibler Folienabschnitt
- 8: unterer flexibler Folienabschnitt
- 9: oberer Schutzabschnitt
- 10: unterer Schutzabschnitt
- 11: proximale Naht
- 12: distale Enden der flexiblen Folienabschnitte
- 13: proximales Ende des unteren flexiblen Folienabschnitts
- 14: distale Richtung
- 15: erster Verbindungsort
- 16: proximales Ende des oberen flexiblen Folienabschnitts
- 17: zweiter Verbindungsort
- 18: Mitnehmer
- 19: Stifte
- 20: proximale Richtung
- 21: Ausnehmungen
- 22: proximales Ende des unteren Schutzabschnitts
- 23: Testfeld
- 24: distale Enden der Schutzabschnitte
- 25: Halteelement
- 26: Distanzstück
- 27: proximales Ende des oberen Schutzabschnitts
- 28: Biegerichtung
- 29: proximale Öffnung
- 30: erster seitlicher Rand
- 31: zweiter seitlicher Rand
- 32: erstes Loch
- 33: zweites Loch

## Patentansprüche

1. Medizinisches Hilfsmittel, enthaltend eine Lanzette (2) mit einem proximalen (5) und einem distalen Ende, wobei die Lanzette (2) einen Lanzettenkörper (4) und an dem proximalen Ende (5) eine Lanzettenspitze (3) aufweist, und wobei die Lanzette (2) zumindest teilweise von einer Verpackung umschlossen ist, die mindestens einen flexiblen Folienabschnitt (7, 8) umfasst, der ein distales Ende (12), ein proximales Ende (16), einen ersten seitlichen Rand und einen zweiten seitlichen Rand aufweist,
wobei
die von der Verpackung zumindest teilweise umschlossene Lanzette (2) verschiebbar zwischen einem oberen und einem unteren Schutzabschnitt (9, 10) angeordnet ist,
• wobei der mindestens eine flexible Folienabschnitt (7, 8) entlang zumindest eines Segments seiner ersten und zweiten seitlichen Ränder lösbar mit einem weiteren flexiblen Folienabschnitt (7, 8) oder einem der Schutzabschnitte (9, 10) verbunden ist, und
• wobei der mindestens eine flexible Folienabschnitt (8) im Bereich der Lanzettenspitze (3) mit einem weiteren flexiblen Folienabschnitt (7) oder mit einem der Schutzabschnitte (9,10) über eine lösbare Verbindung verbunden ist, so dass die Lanzettenspitze (3) durch die Verpackung umschlossen ist, und
• wobei der mindestens eine flexible Folienabschnitt (7, 8) in einem Abstand zu der lösbaren Verbindung mit einem der Schutzabschnitte (9, 10) über eine feste Verbindung verbunden ist, und **dadurch gekennzeichnet, dass**
• die proximalen Enden (13, 16) des unteren und oberen Schutzabschnitts (10, 9) auseinander biegbar sind, wodurch der Abstand zwischen der lösbaren und der festen Verbindung vergrößerbar und dadurch eine Zugkraft auf die lösbare Verbindung ausübbar ist, so dass ein Öffnen der Verpackung an der lösbaren Verbindung erfolgt und eine proximale Öffnung entsteht, durch die zumindest ein Teil der Lanzette (2) in proximaler Richtung (20) aus der Verpackung herausschiebbar ist.

2. Medizinisches Hilfsmittel gemäß Anspruch 1, **dadurch gekennzeichnet, dass** die Lanzette (2) zumindest teilweise von einer flexiblen Verpackung (6) umschlossen ist, die
• einen oberen flexiblen Folienabschnitt (7) umfasst, der ein distales Ende (12), ein proximales Ende (16), einen ersten seitlichen Rand und einen zweiten seitlichen Rand aufweist, und
• einen unteren flexiblen Folienabschnitt (8) umfasst, der ein distales Ende (12), ein proximales Ende (13), einen ersten seitlichen Rand und einen zweiten seitlichen Rand aufweist,
wobei der obere und der untere flexible Folienabschnitt (7, 8) entlang zumindest eines Segments ihrer ersten und zweiten seitlichen Ränder lösbar miteinander verbunden sind und die Lanzette (2) zumindest teilweise umschließen, wobei der untere flexible Folienabschnitt (8) und der obere flexible Folienabschnitt (7) vor der Lanzettenspitze (3) über eine proximale Naht (11) lösbar miteinander verbunden sind, wodurch die Lanzettenspitze (3) durch die flexible Verpackung (6) umschlossen ist, und wobei das proximale Ende (13) des unteren flexiblen Folienabschnitts (8) fest mit dem unteren Schutzabschnitt (10) und das proximale Ende (16) des oberen flexiblen Folienabschnitts (7) fest mit dem oberen Schutzabschnitt (9) verbunden ist und zum Öffnen der flexiblen Verpackung (6) an der proximalen Naht (11) die proximalen Enden (13, 16) des unteren und oberen Schutzabschnitts (10, 9) auseinander biegbar sind, woraufhin zumindest ein Teil der Lanzette (2) aus der flexiblen Verpackung (6) und den Schutzabschnitten (9, 10) in proximaler Richtung (20) herausschiebbar ist.

3. Medizinisches Hilfsmittel gemäß Anspruch 2, **dadurch gekennzeichnet, dass** die oberen und unteren flexiblen Folienabschnitte (7, 8) Abschnitte einer einzigen flexiblen Folie sind, die an den distalen Enden (12) der flexiblen Folienabschnitte (7, 8) umgebogen ist.

4. Medizinisches Hilfsmittel gemäß einem der Ansprüche 1 bis 3, **dadurch gekennzeichnet, dass** die Lanzette (2) eine durch einen halboffenen Kanal gebildete Kapillarstruktur aufweist.

5. Medizinisches Hilfsmittel gemäß einem der Ansprüche 1 bis 4, enthaltend einen durch zumindest einen der Schutzabschnitte (9, 10) hindurch greifenden Mitnehmer (18), der mit dem Lanzettenkörper (4) verbunden und gemeinsam mit der Lanzette (2) in proximaler Richtung (20) verschiebbar ist.

6. Medizinisches Hilfsmittel gemäß Anspruch 5, **dadurch gekennzeichnet, dass** in zumindest einem der Schutzabschnitte (9, 10) eine Ausnehmung (21) vorgesehen ist, durch die der Mitnehmer (18) beim Verschieben gleitet und geführt wird.

7. Medizinisches Hilfsmittel gemäß einem der Ansprüche 1 bis 6, **dadurch gekennzeichnet, dass** der Lanzettenkörper (4) am distalen Ende in die Verpackung eingeklebt ist.

8. Medizinisches Hilfsmittel gemäß einem der Ansprüche 1 bis 7, **dadurch gekennzeichnet, dass** das proximale Ende (22, 27) einer der Schutzabschnitte (9, 10) innen ein Testfeld (23) trägt, das zur Analyse einer Probe vorgesehen ist.

9. Medizinisches Hilfsmittel gemäß einem der Ansprüche 1 bis 8, **dadurch gekennzeichnet, dass** die distalen Enden (24) der Schutzabschnitte (9, 10) mit einem Halteelement (25) verbunden sind, das ein Distanzstück (26) enthält, mittels dessen die distalen Enden (24) der Schutzabschnitte (9, 10) in einem definierten Abstand zueinander gehalten werden.

10. Verfahren zum Entpacken eines medizinischen Hilfsmittels gemäß einem der Ansprüche 1 bis 9, **gekennzeichnet durch**
• das Auseinanderbiegen der proximalen Enden (22, 27) des unteren und oberen Schutzabschnitts(8, 9), wodurch die Verpackung an der lösbaren Verbindung geöffnet wird, so dass die proximale Öffnung (29) entsteht und
• das Herausschieben zumindest eines Teils der Lanzette (2) aus der Verpackung **durch** die proximale Öffnung (29) und aus den Schutzabschnitten (8, 9) in proximaler Richtung (20), so dass zumindest die Lanzettenspitze (3) über die proximalen Enden (22, 27) der Schutzabschnitte (8, 9) hinausragt.

11. Verwendung eines medizinischen Hilfsmittels gemäß einem der Ansprüche 1 bis 9 zum Aufnehmen einer Probe auf ein innen am proximalen Ende (22, 27) einer der Schutzabschnitte (8, 9) angeordnetes Testfeld (23) durch Aufnehmen der Probe in eine durch einen halboffenen Kanal gebildete Kapillarstruktur der Lanzette (2) und Abstreifen der Probe auf das Testfeld (23).

12. Sensor zum Nachweis mindestens eines Analyten in einer Probe, insbesondere zum Nachweis von Glucose in einer Blutprobe oder in interstitieller Flüssigkeit, enthaltend mindestens ein Testelement mit einem proximalen und einem distalen Ende, wobei das Testelement zumindest teilweise von einer Verpackung umschlossen ist, die mindestens einen flexiblen Folienabschnitt (7, 8) umfasst, der ein distales Ende (12), ein proximales Ende (16), einen ersten seitlichen Rand und einen zweiten seitlichen Rand aufweist, wobei
das von der Verpackung zumindest teilweise umschlossene Testelement verschiebbar zwischen einem oberen und einem unteren Schutzabschnitt (9, 10) angeordnet ist,
• wobei der mindestens eine flexible Folienabschnitt (7, 8) entlang zumindest eines Segments seiner ersten und zweiten seitlichen Ränder lösbar mit einem weiteren flexiblen Folienabschnitt (7, 8) oder einem der Schutzabschnitte (9, 10) verbunden ist, und
• wobei der mindestens eine flexible Folienabschnitt (8) im Bereich des proximalen Endes des Testelements mit einem weiteren flexiblen Folienabschnitt (7) oder mit einem der Schutzabschnitte (9,10) über eine lösbare Verbindung verbunden ist, so dass das proximale Ende des Testelements durch die Verpackung umschlossen ist, und
• wobei der mindestens eine flexible Folienabschnitt (7, 8) in einem Abstand zu der lösbaren Verbindung mit einem der Schutzabschnitte (9, 10) über eine feste Verbindung verbunden ist, und **dadurch gekennzeichnet, dass**
• die proximalen Enden (13, 16) des unteren und oberen Schutzabschnitts (10, 9) auseinander biegbar sind, wodurch der Abstand zwischen der lösbaren und der festen Verbindung vergrößerbar und dadurch eine Zugkraft auf die lösbare Verbindung ausübbar ist, so dass ein Öffnen der Verpackung an der lösbaren Verbindung erfolgt und eine proximale Öffnung entsteht, durch die zumindest ein Teil des Testelements in proximaler Richtung (20) aus der Verpackung herausschiebbar ist.

## Claims

1. Medical aid comprising a lancet (2) with a proximal end (5) and a distal end, the lancet (2) having a lancet body (4) and, at the proximal end (5), a lancet tip (3), and the lancet (2) being at least partially enclosed by a packaging that comprises at least one flexible film section (7, 8) having a distal end (12), a proximal end (16), a first lateral edge and a second lateral edge, the lancet (2), at least partially enclosed by the packaging, being arranged displaceably between an upper protective section (9) and a lower protective section (10),
• wherein the at least one flexible film section (7, 8) is detachably connected, along at least one segment of its first and second lateral edges, to an additional flexible film section (7, 8) or to one of the protective sections (9, 10), and
• wherein the at least one flexible film section (8) is connected to an additional flexible film section (7) or to one of the protective sections (9, 10) in the area of the lancet tip (3) via a detachable connection, such that the lancet tip (3) is enclosed by the packaging, and
• wherein the at least one flexible film section (7, 8) is connected to one of the protective sections (9, 10) via a fixed connection at a distance from the detachable connection, and **characterized in that**
• the proximal ends (13, 16) of the lower and upper protective sections (10, 9) can be bent apart, as a result of which the distance between the detachable connection and the fixed connection can be increased and a tensile force can thus be applied to the detachable connection, such that the packaging is opened at the detachable connection and a proximal opening is created through which at least part of the lancet (2) can be pushed out from the packaging in the proximal direction (20).

2. Medical aid according to Claim 1, **characterized in that** the lancet (2) is at least partially enclosed by a flexible packaging (6) which comprises
• an upper flexible film section (7) having a distal end (12), a proximal end (16), a first lateral edge and a second lateral edge, and
• a lower flexible film section (8) having a distal end (12), a proximal end (13), a first lateral edge and a second lateral edge,
wherein the upper and lower flexible film sections (7, 8) are detachably connected to each other along at least one segment of their first and second lateral edges and at least partially enclose the lancet (2), wherein the lower flexible film section (8) and the upper flexible film section (7) are detachably connected to each other via a proximal seam (11) in front of the lancet tip (3), and the lancet tip (3) is thus enclosed by the flexible packaging (6), and wherein the proximal end (13) of the lower flexible film section (8) is fixedly connected to the lower protective section (10) and the proximal end (16) of the upper flexible film section (7) is fixedly connected to the upper protective section (9), and the proximal ends (13, 16) of the lower and upper protective sections (10, 9) can be bent apart in order to open the flexible packaging (6) at the proximal seam (11), whereupon at least part of the lancet (2) can be pushed out from the flexible packaging (6) and from the protective sections (9, 10) in the proximal direction (20).

3. Medical aid according to Claim 2, **characterized in that** the upper and lower flexible film sections (7, 8) are sections of a single flexible film that is bent back at the distal ends (12) of the flexible film sections (7, 8).

4. Medical aid according to one of Claims 1 to 3, **characterized in that** the lancet (2) has a capillary structure formed by a half-open channel.

5. Medical aid according to one of Claims 1 to 4, comprising a driver element (18) which engages through at least one of the protective sections (9, 10), is connected to the lancet body (4) and can be displaced together with the lancet (2) in the proximal direction (20).

6. Medical aid according to Claim 5, **characterized in that** at least one of the protective sections (9, 10) has a recess (21) through which the driver element (18) slides and is guided during its displacement.

7. Medical aid according to one of Claims 1 to 6, **characterized in that** the distal end of the lancet body (4) is adhesively bonded into the packaging.

8. Medical aid according to one of Claims 1 to 7, **characterized in that** the proximal end (22, 27) of one of the protective sections (9, 10) has, on its inside, a test area (23), which is provided for analysis of a sample.

9. Medical aid according to one of Claims 1 to 8, **characterized in that** the distal ends (24) of the protective sections (9, 10) are connected to a holding element (25) that comprises a spacer piece (26), by means of which the distal ends (24) of the protective sections (9, 10) are held at a defined distance from each other.

10. Method for unpacking a medical aid according to one of Claims 1 to 9, **characterized by**
• bending the proximal ends (22, 27) of the lower and upper protective sections (9, 10) apart, as a result of which the packaging is opened at the detachable connection, such that the proximal opening (29) is created, and
• pushing at least part of the lancet (2) out from the packaging and from the protective sections (9, 10) in the proximal direction (20) through the proximal opening (29), such that at least the lancet tip (3) protrudes beyond the proximal ends (22, 27) of the protective sections (9, 10).

11. Use of a medical aid according to one of Claims 1 to 9 for taking up a sample on a test area (23) arranged on the inside at the proximal end (22, 27) of one of the protective sections (9, 10), by means of the sample being taken up in a capillary structure formed by a half-open channel of the lancet (2) and by means of the sample being scraped off onto the test area (23).

12. Sensor for detection of at least one analyte in a sample, in particular for detection of glucose in a blood sample or in interstitial fluid, comprising at least one test element with a proximal end and a distal end, the test element being at least partially enclosed by a packaging that comprises at least one flexible film section (7, 8) having a distal end (12), a proximal end (16), a first lateral edge and a second lateral edge,
the test element, at least partially enclosed by the packaging, being arranged displaceably between an upper protective section (9) and a lower protective section (10),
• wherein the at least one flexible film section (7, 8) is detachably connected, along at least one segment of its first and second lateral edges, to an additional flexible film section (7, 8) or to one of the protective sections (9, 10), and
• wherein the at least one flexible film section (8) is connected to an additional flexible film section (7) or to one of the protective sections (9, 10) in the area of the proximal end of the test element via a detachable connection, such that the proximal end of the test element is enclosed by the packaging, and
• wherein the at least one flexible film section (7, 8) is connected to one of the protective sections (9, 10) via a fixed connection at a distance from the detachable connection, and **characterized in that**
• the proximal ends (13, 16) of the lower and upper protective sections (10, 9) can be bent apart, as a result of which the distance between the detachable connection and the fixed connection can be increased and a tensile force can thus be applied to the detachable connection, such that the packaging is opened at the detachable connection and a proximal opening is created through which at least part of the test element can be pushed out from the packaging in the proximal direction (20).

## Revendications

1. Dispositif médical, comprenant une lancette (2) présentant une extrémité proximale (5) et une extrémité distale, dans lequel la lancette (2) présente un corps de lancette (4) et une pointe de lancette (3) à l'extrémité proximale (5), et dans lequel la lancette (2) est enveloppée au moins partiellement par un emballage, qui comprend au moins une partie de feuille flexible (7, 8) qui présente une extrémité distale (12), une extrémité proximale (16), un premier bord latéral et un deuxième bord latéral, dans lequel la lancette (2) enveloppée au moins partiellement par l'emballage est disposée de façon coulissante entre une partie de protection supérieure et une partie de protection inférieure (9, 10),
• dans lequel ladite au moins une partie de feuille flexible (7, 8) est assemblée le long d'au moins un segment de ses premier et deuxième bords latéraux de façon séparable à une autre partie de feuille flexible (7, 8) ou à une des parties de protection (9, 10), et
• dans lequel ladite au moins une partie de feuille flexible (8) est assemblée dans la région de la pointe de lancette (3) à une autre partie de feuille flexible (7) ou à une des parties de protection (9, 10) au moyen d'un assemblage séparable, de telle manière que la pointe de lancette (3) soit enveloppée par l'emballage, et
• dans lequel ladite au moins une partie de feuille flexible (7, 8) est assemblée, à distance de l'assemblage séparable, à une des parties de protection (9, 10) au moyen d'un assemblage fixe, et
**caractérisé en ce que**
• les extrémités proximales (13, 16) des parties de protection inférieure et supérieure (10, 9) peuvent s'écarter en se courbant, ce qui permet d'augmenter la distance entre l'assemblage séparable et l'assemblage fixe et ainsi d'exercer une force de traction sur l'assemblage séparable, de telle manière qu'il se produise une ouverture de l'emballage à l'assemblage séparable et qu'il se forme une ouverture proximale, par laquelle au moins une partie de la lancette (2) peut être retirée hors de l'emballage dans la direction proximale (20) .

2. Dispositif médical selon la revendication 1, **caractérisé en ce que** la lancette (2) est enveloppée au moins partiellement par un emballage flexible (6), qui
• comprend une partie de feuille flexible supérieure (7), qui présente une extrémité distale (12), une extrémité proximale (16), un premier bord latéral et un deuxième bord latéral, et
• comprend une partie de feuille flexible inférieure (8), qui présente une extrémité distale (12), une extrémité proximale (13), un premier bord latéral et un deuxième bord latéral,
dans lequel les parties de feuille flexible supérieure et inférieure (7, 8) sont assemblées l'une à l'autre d'une façon séparable le long d'au moins un segment de leurs premier et deuxième bords latéraux et enveloppent au moins partiellement la lancette (2), dans lequel la partie de feuille flexible inférieure (8) et la partie de feuille flexible supérieure (7) sont assemblées l'une à l'autre d'une façon séparable devant la pointe de lancette (3) au moyen d'une soudure proximale (11), la pointe de lancette (3) étant ainsi enveloppée par l'emballage flexible (6), et dans lequel l'extrémité proximale (13) de la partie de feuille flexible inférieure (8) est assemblée de façon fixe à la partie de protection inférieure (10) et l'extrémité proximale (16) de la partie de feuille flexible supérieure (7) est assemblée de façon fixe à la partie de protection supérieure (9) et les extrémités proximales (13, 16) des parties de protection inférieure et supérieure (10, 9) peuvent s'écarter en se courbant pour ouvrir l'emballage flexible (6) à la soudure proximale (11), au moins une partie de la lancette (2) pouvant de ce fait être ensuite retirée hors de l'emballage flexible (6) et des parties de protection (9, 10) dans la direction proximale (20).

3. Dispositif médical selon la revendication 2, **caractérisé en ce que** les parties de feuille flexible supérieure et inférieure (7, 8) sont des parties d'une seule feuille flexible, qui sont repliées aux extrémités distales (12) des parties de feuille flexible (7, 8).

4. Dispositif médical selon l'une quelconque des revendications 1 à 3, **caractérisé en ce que** la lancette (2) présente une structure capillaire formée par un canal semi-ouvert.

5. Dispositif médical selon l'une quelconque des revendications 1 à 4, comprenant un entraîneur (18) traversant au moins une des parties de protection (9, 10), qui est relié au corps de lancette (4) et qui peut coulisser avec la lancette (2) dans la direction proximale (20).

6. Dispositif médical selon la revendication 5, **caractérisé en ce qu'**il est prévu, dans au moins une des parties de protection (9, 10), un évidement (21) à travers lequel l'entraîneur (18) glisse et est guidé lors du déplacement.

7. Dispositif médical selon l'une quelconque des revendications 1 à 6, **caractérisé en ce que** le corps de lancette (4) est collé dans l'emballage à l'extrémité distale.

8. Dispositif médical selon l'une quelconque des revendications 1 à 7, **caractérisé en ce que** l'extrémité proximale (22, 27) d'une des parties de protection (9, 10) porte intérieurement un champ de test (23), qui est prévu pour l'analyse d'un échantillon.

9. Dispositif médical selon l'une quelconque des revendications 1 à 8, **caractérisé en ce que** les extrémités distales (24) des parties de protection (9, 10) sont assemblées à un élément de maintien (25), qui comprend une pièce d'écartement (26), au moyen de laquelle les extrémités distales (24) des parties de protection (9, 10) sont maintenues à une distance définie l'une de l'autre.

10. Procédé pour déballer un dispositif médical selon l'une quelconque des revendications 1 à 9, **caractérisé par**
• l'écartement par courbure des extrémités proximales (22, 27) des parties de protection inférieure et supérieure (9, 10), l'emballage étant ainsi ouvert à l'assemblage séparable de telle façon que l'ouverture proximale (29) se forme, et
• l'extraction d'au moins une partie de la lancette (2) hors de l'emballage à travers l'ouverture proximale (29) et hors des parties de protection (9, 10) dans la direction proximale (20), de telle manière qu'au moins la pointe de lancette (3) sorte au-delà des extrémités proximales (22, 27) des parties de protection (9, 10).

11. Utilisation d'un dispositif médical selon l'une quelconque des revendications 1 à 9 pour la réception d'un échantillon sur un champ de test (23) disposé intérieurement sur l'extrémité proximale (22, 27) d'une des parties de protection (9, 10), par réception de l'échantillon dans une structure capillaire de la lancette (2), formée par un canal semi-ouvert, et étalement de l'échantillon sur le champ de test (23).

12. Détecteur pour détecter au moins un analyte dans un échantillon, en particulier pour détecter le glucose dans un échantillon de sang ou dans un liquide interstitiel, comprenant au moins un élément de test avec une extrémité proximale et une extrémité distale, dans lequel l'élément de test est enveloppé au moins partiellement par un emballage qui comprend au moins une partie de feuille flexible (7, 8), qui présente une extrémité distale (12), une extrémité proximale (16), un premier bord latéral et un deuxième bord latéral, dans lequel l'élément de test enveloppé au moins partiellement par l'emballage est disposé de façon coulissante entre une partie de protection supérieure et une partie de protection inférieure (9, 10),
• dans lequel ladite au moins une partie de feuille flexible (7, 8) est assemblée le long d'au moins un segment de ses premier et deuxième bords latéraux de façon séparable à une autre partie de feuille flexible (7, 8) ou à une des parties de protection (9, 10), et
• dans lequel ladite au moins une partie de feuille flexible (8) est assemblée dans la région de l'extrémité proximale de l'élément de test à une autre partie de feuille flexible (7) ou à une des parties de protection (9, 10) au moyen d'un assemblage séparable, de telle manière que l'extrémité proximale de l'élément de test soit enveloppée par l'emballage, et
• dans lequel ladite au moins une partie de feuille flexible (7, 8) est assemblée, à distance de l'assemblage séparable, à une des parties de protection (9, 10) au moyen d'un assemblage fixe, et
**caractérisé en ce que**
• les extrémités proximales (13, 16) des parties de protection inférieure et supérieure (10, 9) peuvent s'écarter en se courbant, ce qui permet d'augmenter la distance entre l'assemblage séparable et l'assemblage fixe et ainsi d'exercer une force de traction sur l'assemblage séparable, de telle manière qu'il se produise une ouverture de l'emballage à l'assemblage séparable et qu'il se forme une ouverture proximale, par laquelle au moins une partie de l'élément de test peut être retirée hors de l'emballage dans la direction proximale (20).
